# EUROPEAN PATENT SPECIFICATION

(11) **EP 1 077 668 B1**
(45) Date of publication and mention of the grant of the patent: **19.05.2010**
(21) Application number: 00914846.1
(22) Date of filing: 08.03.2000
(51) Int. Cl.: A61K 8/04, A61K 8/34, A61K 8/73, A61K 8/81, A61K 8/87, A61Q 19/00

(54) **GEL POWDER COMPOSITION**
GEL-PULVER ZUSAMMENSETZUNG
COMPOSITION SOUS FORME DE POUDRE GEL

(30) Priority: 19.03.1999 US 273087
(43) Date of publication of application: 28.02.2001
(73) Proprietor: Color Access, Inc., Melville, New York 11747 (US)
(72) Inventor: SCORDAMAGLIA-CROCKETT, Barbara, A., Greenlawn, NY 11740 (US); HACKFORD, Timothy, W., Stewart Manor, NY 11530 (US)
(74) Representative: Hirsch & Associés
(86) International application number: PCT/US2000/005927
(87) International publication number: WO 2000/056272

(56) References cited:
- EP-A- 0 614 656
- EP-A- 0 676 195
- WO-A-94/14403
- WO-A-99/06012
- US-A- 4 664 909
- US-A- 5 034 216
- US-A- 5 626 856
- US-A- 5 658 577
- DATABASE WPI Week 198847 Derwent Publications Ltd., London, GB; AN 1988-333821 XP002141092 & JP 63 246308 A (LION CORP), 13 October 1988 (1988-10-13)
- DATABASE WPI Week 199819 Derwent Publications Ltd., London, GB; AN 1998-212690 XP002141093 & JP 10 059817 A (SHISEIDO CO. LTD.), 3 March 1998 (1998-03-03)
- DATABASE WPI Week 199850 Derwent Publications Ltd., London, GB; AN 1998-584154 (50) XP002141094 & BR 9 701 247 A (MAIA W.J.), 10 November 1998 (1998-11-10)
- DATABASE WPI Week 199345 Derwent Publications Ltd., London, GB; AN 1993-357115 XP002141095 & JP 05 262622 A (NEGAMI KOGYO KK), 12 October 1993 (1993-10-12)
- DATABASE WPI Week 200010 Derwent Publications Ltd., London, GB; AN 2000-111583 XP002141096 & JP 11 349442 A (POLA CHEM. IND. INC.), 21 December 1999 (1999-12-21)
- DATABASE WPI Week 200008 Derwent Publications Ltd., London, GB; AN 2000-092590 XP002141097 & JP 11 335259 A (SHINETSU CEM. IND. CO. LTD.), 7 December 1999 (1999-12-07)

## Description

### Field of the Invention

The invention relates to cosmetic compositions. More specifically, the invention relates to gel powder compositions with improved feel on application to skin.

### Background of the Invention

Liquid powder compositions have become very popular in recent years for a number of reasons. Such compositions have an advantage over the traditional dusting powders in the mode of delivery is more precise; with a standard dusting powder, the user was forced to sprinkle the product over the areas where powder application was desired. Because of the light weight and particulate nature of powders generally, however, precise and limited application of the powder to the body was difficult. With liquid powders, however, the user simply smoothes the liquid product on the part of the body on which powder application is desired, and after a short time, the liquid evaporates, leaving behind the powder only where it is needed. Generally speaking, liquid powders are intended to be initially refreshing and smoothing to the skin, with the liquid base eventually transforming to a silky powder on the skin. Unlike other cosmetics that utilize powder components, for example mica, or other fillers used in color cosmetics, the user is intended to feel the powder on the skin, and to that end the powder components tend to be of a larger particle size than in other powder-containing cosmetics.

Liquid powders come in a variety of forms. All have in common the presence of a particulate powdery material combined with at least one volatile component. For example, US Patent Nos. 5,338,535 and 5,626,856 disclose non-aqueous liquid powders containing a volatile cyclomethicone combined with particulate carbohydrates such as cornstarch or tapioca. Non-aqueous compositions, however, can have a heavy or greasy feel when being applied initially, and thus may be less desirable for individuals with oily skin. Aqueous or alcoholic powder products are also known. These provide the advantage of a non-oily base, and thus can be more refreshing and cooling to apply and wear than oil-based, non-aqueous products. In such non-oily products, alcohol, water, or more typically, alcohol combined with water provide the volatile component, and the powder utilized is again primarily a starch such as tapioca or cornstarch.

US Patent No. 4,664, 909 discloses fast drying, pituitous compositions containing water, alcohol, gelling agent and micropulverized sodium bicarbonate or zinc undecylate.

In the majority of liquid powders, the particulate carbohydrates are the traditional powder components. This is primarily due to the cost of the materials, which is fairly low. Although effective in providing a powdery feel, however, these components can often leave a gritty uneven feeling on the skin when the volatile evaporates off. There thus continues to be a need for a liquid powder that provides the desirable cool feeling upon application and dry down, but which leaves a silky, smooth feeling on the skin when only the powder component remains.

### Summary of the Invention

The present invention relates to an aqueous liquid gel powder composition according to claim 1. In a preferred embodiment, the spherical microparticle is a silicone resin, a polyurethane particle, or a combination of both.

### Detailed Description of the Invention

The composition of the invention provides a cooling liquid powder composition that leaves a very smooth, powdery but non-gritty feeling on the skin when the volatile portion has evaporated off. It has been unexpectedly discovered that incorporation of spherical microparticles as a portion of the powder phase of an otherwise standard aqueous liquid powder composition confers a substantially different feel to the final product on the skin. Unlike the previously employed particulate carbohydrates, the particles of the invention are regularly spherical, and have a relatively uniform particle size distribution. In a preferred embodiment, the average particle diameter of the microspheres ranges from 1-20µ. Particles of this type can be of a variety of chemical compositions, for example, nylon, silica, silicone resin, polyethylene, polyurethane, and acrylate or methacrylate polymers or copolymers. Particularly preferred among these microspheres are polyurethane and silicone resin microspheres. Examples of commercially available resins of these types are BPD-500, a polyurethane(HDI/trimethylyol hexyl lactone) microsphere, and Tospearl silicone resin (polymethylsilsesquioxane) microspheres, both available from Kobo Products, Inc., South Plainfield, New Jersey. Particularly preferred of the Tospearl microspheres is Tospearl 145, having an average particle diameter of about 4.5µ. Similar microspheres are also available from Presperse(Piscataway, NJ).

The powder component of the composition comprises at least one type of spherical microparticle, but may also comprise a combination of microspheres. Overall, the powder component comprises 5-20% by weight of the total composition. The microspheres are a portion of the powder component; in other words, the spherical microparticles are combined with a starch powder component. It has been unexpectedly discovered that a very desirable, soft, silky product can be obtained by the combination of the microspheres with a starch bulking agent or powder component. Examples of useful starch bulking agents are particulate starches, such as tapioca starch, corn starch, rice starch, potato starch, or wheat starch. Other useful bulking agents, particularly for non-aqueous products are amylopectin, agar, cellulose or a cellulose ether, glycogen, algin or carrageenan. Silica, nylon, polyethylene, polypropylene, mica, or talc could also be used. Starches are particularly preferred for aqueous gel powders. Particularly surprising is that even a relatively small proportion of microspheres can substantially improve the feel of the final product. Preferably, the starch and microspheres are present in a ratio of 5:1 to 1:5(starch to microspheres), more preferably 3:1 to 1.5:1. In fact, although a product containing all microspheres as powder component exhibits a silky powdery feel superior to that of an identical product containing all starch, a product containing a mixture of microspheres and starch exhibits an even better after-feel, and initially goes on more smoothly, with less tackiness, than either of the other products. In addition, the combination of starch and microspheres permits the formulation of an enhanced product with relatively little increase in expense over the product containing only starch.

The carrier is a gel base, which permits the suspension the powder and microparticle components. The base, is an aqueous gel including a volatile component which is a C₁ to C₄ monohydric alcohol. The gelling agent can be any which is appropriate for gelling an aqueous base, and includes, but is not limited to, carbomer, hydroxypropyl methylcellulose, hydroxyethylcellulose, hydroxy propyl guar, hydroxypropyl cellulose, or potato starch modified. Particularly preferred is an acrylates/C10-C30 alkyl acrylates crosspolymer, commercially available under the tradename Pemulen TR-2 from (B.F. Goodrich Specialty Chemicals, Cleveland, Ohio).

Although less preferred, the base may also contain an oil component. A preferred oil comprises a volatile oil, such as volatile cyclic and linear silicones, for example, cyclomethicone; or straight or branched chain hydrocarbons having from 8-20 carbon atoms, such as decane, dodecane, tridecane, tetradecane, and C8-20 isoparaffins. A preferred volatile oil is cyclomethicone. Useful oil gellants include, but are not limited to, organopolysiloxane elastomers, ethylene/methacrylic acid copolymer, ethylene/acrylic acid copolymer, or polyethylene.

Generally speaking, the liquid base component will constitute from about 10 to about 90%, preferably about 20 to about 60%, more preferably from about 30-50%, by weight of the total composition. The amount of gellant will vary depending upon the identity of the gellant, and the desired texture of the final product, but will be in the range of from about 0.1 to about 5% of the total composition.

The alcohol component is a short chain, i.e., C1-C4 monohydric alcohol, such as methanol, ethanol or isopropanol. The alcohol acts as a volatile to facilitate the rapid drying of the product. The alcohol component is present in an amount of from 20-70% by weight.

There may be other optional components forming part of the composition. Since the liquid powder composition can be used for a variety of purposes, and not just as a body powder, it may be desirable to add additional components depending on the intended end use. In some cases, the desired components may not necessarily be soluble in an aqueous base. In such a case, it will be desirable that the alcohol component assists in solubilizing such components.

In a particularly preferred embodiment, the composition is used as a body powder, preferably a fragranced body powder. In such a composition, fragrance will normally be added in an amount of from about 0.1-6% by weight of the composition. Such compositions can also contain standard cosmetic ingredients, such as colorants, preservatives, emulsifiers, skin conditioners, emollients, and the like. In addition to this embodiment, however, as noted above, the liquid powder composition can also be used for purposes other than a body powder. For example, it may be used as a delivery system to provide both cosmetic and pharmaceutical actives to the skin. Thus, in addition to the essential components of the invention, the composition can also comprise one or more active components. Examples of such active agents which may form part of the composition include, but are not limited to, those that improve or eradicate age spots, keratoses and wrinkles, analgesics, anesthetics, anti-acne agents, antibacterials, antiyeast agents, antifungal agents, antiviral agents, antidandruff agents, antidermatitis agents, antipruritic agents, antiemetics, antimotion sickness agents, anti-inflammatory agents, antihyperkeratolytic agents, anti-dry skin agents, antiperspirants, antipsoriatic agents, antiseborrheic agents, antiaging agents, antiwrinkle agents, antiasthmatic agents and bronchodilators, sunscreen agents, antihistamine agents, skin lightening agents, depigmenting agents, wound-healing agents, vitamins, corticosteroids, self-tanning agents, antioxidants, free-radical scavengers, or hormones. More specific examples of useful active agents include retinoids, topical cardiovascular agents, clotrimazole, ketoconazole, miconozole, griseofulvin, hydroxyzine, diphenhydramine, pramoxine, lidocaine, procaine, mepivacaine, monobenzone, erythromycin, tetracycline, clindamycin, meclocyline, hydroquinone, minocycline, naproxen, ibuprofen, theophylline, cromolyn, albuterol, retinol, retinoic acid, 13-cis retinoic acid, hydrocortisone, hydrocortisone 21-acetate, hydrocortisone 17-valerate, hydrocortisone 17-butyrate, betamethasone valerate, betamethasone diproprionate, triamcinolone acetonide, fluocinonide, clobetasol, proprionate, benzoyl peroxide, crotamiton, propranolol, promethazine, vitamin A palmitate, vitamin E acetate, DHEA and derivatives thereof, alpha- or beta-hydroxy acids, and mixtures thereof. The amount of active agent to be used in any given formulation is readily determined in accordance with its usual dosage.

The invention is further illustrated by the following non-limiting examples:

### EXAMPLES

### Example I.

The following formula represents a fragranced body powder prepared according to the invention:

| Material | Weight Percent |
|---|---|
| | |

| Phase I | |
|---|---|
| water | QS |
| benzophenone-4 | 0.30 |
| | |

| Phase II | |
|---|---|
| acrylates/C10-C30 alkyl acrylate | |
| crosspolymer | 0.45 |
| | |

| Phase III | |
|---|---|
| octyl methoxycinnamate | 0.10 |
| isostearyl neopentanoate | 1.00 |
| tocopheryl acetate | 0.05 |
| | |

| Phase IV | |
|---|---|
| tapioca starch | 12.00 |
| polymethylsilsesquioxane | 4.00 |
| HDI/trimethylol hexylactone | |
| crosspolymer | 3.00 |
| | |

| Phase V | |
|---|---|
| fragrance | 3.00 |
| denatured alcohol | 30.00 |
| | |

| Phase VI | |
|---|---|
| phenoxyethanol | 0.70 |
| stearyl heptanoate | 3.00 |
| dimethicone copolyol | 0.75 |
| | |

| Phase VII | |
|---|---|
| cyclomethicone/dimethicone copolyol | 1.25 |

The materials of Phase III are premixed under propeller agitation while heating to 60°C, and mixed until all components are dissolved. Phase II is sprinkled into Phase III materials until uniformly dispersed. In a separate kettle, Phase I materials are mixed under propeller agitation until all solids are dissolved. The combined Phases II and III are added to Phase I, and the gellant allowed to hydrate for 30 minutes. A premix of phase VI materials is made, and then added to the materials in the kettle while propeller mixing and heating to 50°C, then mixing until all solids are dissolved. Phase V materials are separately mixed until uniform. After a 30 minute mix, the Phase IV powders are sprinkled into the main kettle and mixed until all powders are uniformly dispersed. The Phase V materials are then added to the main kettle with propeller mixing. Finally, Phase VII is added under side-swipe agitation.

### Example II

Three liquid powder products are compared to determine consumer's perceptions of some key aesthetic characteristics. The three products compared were: (T&S) a product containing both spherical powders and tapioca, as described in Example I; (T)a product containing only tapioca; and (S)a product containing only spherical powders. Each product is tested with a total of 52 females ages 18-60, with normal or normal/oily body skin, and who are regular users of a body lotion or body powder. The panelists are instructed to smooth the product all over the body at least once a day and not to use any other body lotions or body powders during the test period. Each product is tested for a period of two weeks, after which a self-administered questionnaire is answered by the panelist.

With respect to the characteristic of product texture, both spherical powder containing products are found by the panelists to be more soft, smooth and silky than the product containing only tapioca. The product containing both tapioca and spherical powders performs best of all the tested products in this category(extremely/very soft/smooth/silky: T&S87% vs. T69% vs. S81%). Similarly, both spherical powder-containing products received higher ratings on the measures of positive purchase intent(definitely/probably would buy:T&S73% vs. T56% vs. S65%) and overall ratings (excellent/very good:T&S:73% vs. T54% vs. S65%), with the tapioca and spherical powder product receiving the highest ratings in all these categories.

## Claims

1. An aqueous liquid powder gel composition comprising an aqueous base including a volatile component comprising from 20 to 70% by weight of a C1 to C4 monohydric alcohol component, from 0.1 to 5% by weight of a gelling agent and from 5 to 20% by weight of a powder component, the powder component comprising at least one cosmetically acceptable spherical microparticle and a starch, wherein the weights are based on the total weight of the gel composition, and wherein the gel composition is liquid when applied to the skin, and dries to a powder on the skin after application.

2. The gel composition of claim 1 wherein the starch and microspheres are present in a ratio of 5:1 to 1:5 (starch to microspheres), more preferably 3:1 to 1.5:1.

3. The gel composition of claim 1 or 2 comprising a microparticle selected from the group consisting of nylon, silica, silicone resin, polyethylene, polyurethane, and acrylate or methacrylate polymers or copolymers.

4. The gel composition of claims 1 or 2 in which the average particle size diameter of the microparticle is 1 to 20µ.

5. The gel composition of any of claims 1-4 in which the powder component comprises polyurethane microparticles

6. The gel composition of any of the preceding claims in which the powder component comprises silicone resin microparticles.

7. The gel composition of any of claims 1-6 which comprises a gelling agent selected from the group consisting of carbomer, hydroxypropyl methylcellulose, hydroxyethylcellulose, hydroxy propyl guar, hydroxypropyl cellulose, potato starch modified, and acrylates/C 1 0-C30 alkyl acrylates crosspolymer.

8. The gel composition of any of claims 1 to 7, in which the starch is selected from the group consisting of tapioca starch, corn starch, rice starch, potato starch, and wheat starch.

9. The gel composition of claim 8 which comprises an aqueous base; and acrylates/C 10-C30 alkyl acrylates crosspolymer; and a powder component comprising microparticles selected from the group consisting of polyurethane microparticles, silicone resin microparticles and combinations thereof combined with a tapioca or corn starch.

10. The gel composition of claim 8 which comprises from 10-90% by weight of an aqueous base; and from 0.1 to 5% by weight of an acrylates/C10-30 alkyl acrylates crosspolymer; wherein said weights are based on the total weight of the gel composition, the powder component comprising microparticles selected from the group consisting of polyurethane microparticles, silicon resin microparticles and a combination thereof combined with a starch.

11. The gel composition of claim 10 in which the powder component comprises 0.1-5% by weight polyurethane microparticles, 0.1-5% by weight silicone resin microparticles, and 1-20% by weight tapioca starch, wherein said weights are based on the total weight of the gel composition.

12. The gel composition of any of claims 1-7 in which the powder component comprises spherical microparticles selected from the group consisting of polyurethane microparticles, silicone resin microparticles and a combination thereof, and a starch selected from the group consisting of tapioca and cornstarch.

## Patentansprüche

1. Eine wässrige, flüssige Pulvergelzusammensetzung die eine wasserhaltige Basis umfasst mit einer volatilen Komponenten von 20 bis 70 Gew.% einer C1 bis C4 einwertigen Alkohol Komponenten, von 0,1 bis 5 Gew.% eines Geliermittels und von 5 bis 20 Gew.% einer Pulverkomponente, wobei die Pulverkomponente wenigstens eine kosmetisch akzeptierbare sphärische Mikropartikel und eine Stärke umfasst, wobei die Gewichte auf dem Gesamtgewicht der Gelzusammensetzung basiert sind, und wobei die Gelzusammensetzung beim Auftragen auf die Haut flüssig ist und nach dem Auftragen zu einem Pulver trocknet.

2. Die Gelzusammensetzung nach Anspruch 1, in der die Stärke und Mikrokugeln in einem Verhältnis von 5:1 (Stärke zu Mikrokugeln) vorzugsweise von 3:1 bis 1,5:1 vorhanden sind.

3. Die Gelzusammensetzung nach Anspruch 1 oder 2, welche einen Mikropartikel enthält, der aus der Gruppe bestehend aus Nylon, Silika, Silikonharz, Polyethylen, Polyurethan, und Acryl- oder Methacryl- Polymeren oder Copolymeren ausgewählt ist.

4. Die Gelzusammensetzung nach Ansprüchen 1 oder 2, in der der mittlere Größendiameter der Mikropartikel 1 bis 20µ beträgt.

5. Die Gelzusammensetzung nach einem der Ansprüche 1-4, in der die Pulverkomponente Polyurethan Mikropartikel enthält.

6. Die Gelzusammensetzung nach einem der vorhergehenden Ansprüche in welcher die Pulverkomponente Silikonharzmikropartikel enthält.

7. Die Gelzusammensetzung nach einem der Ansprüche 1-6, die einen Gelbildner umfasst der ausgewählt ist aus der Gruppe bestehend aus Carbomer, Hydroxypropylmethylzellulose, Hydroxyethylzellulose, Hydroxypropylguargummi, Hydroxypropylzellulose, modifizierter Kartoffelstärke, und Acrylate/C10-C30 Alkylacrylat Kreuzpolymere umfasst.

8. Die Gelzusammensetzung nach einem der Ansprüche 1 bis 7, in der die Stärke ausgewählt ist aus der Gruppe bestehend aus Tapiokastärke, Komstärke, Reisstärke, Kartoffelstärke, und Weizenstärke.

9. Die Gelzusammensetzung nach Anspruch 8, welche eine wässerige Basis enthält; und Acrylate/C10-C30 Alkylacrylate Kreuzpolymer; und eine Pulverkomponente die Mikropartikel enthält die aus der Gruppe bestehend aus Polyurethanmikropartikeln, Silikonharzmikropartikeln und deren Kombination mit Tapioka oder Komstärke, ausgewählt sind.

10. Die Gelzusammensetzung nach Anspruch 8, welche von 10-90 Gew.% einer wässerigen Basis enthält; und 0,1 bis 5 Gew% Acrylate/C10-C30 Alkyl Acrylate Kreuzpolymer; wobei die Gewichte auf dem Gesamtgewicht der Gelzusammensetzung basiert sind, die Pulverkomponente Mikropartikel enthält, welche aus der Gruppe bestehend aus Polyurethanmikropartikeln, Silikonharzmikropartikeln und deren Kombination mit einer Stärke, ausgewählt sind.

11. Die Gelzusammensetzung nach Anspruch 10, in der die Pulverkomponente 0,1-5 Gew.% Polyurethanmikropartikel, 0,1-5 Gew.% Silikonharzmikropartikel, und 1-20 Gew.% Tapiokastärke enthält, wobei diese Gewichte auf dem Gesamtgewicht der Gelzusammensetzung basiert sind.

12. Die Gelzusammensetzung nach einem der Ansprüche 1-7, in der die Pulverkomponente sphärische Mikropartikel enthält die aus der Gruppe bestehend aus Polyurethanmikropartikeln, Silikonharzmikropartikeln und deren Kombination, und einer Stärke aus der Gruppe bestehend aus Tapiokastärke und Komstärke, ausgewählt sind.

## Revendications

1. Une composition de gel pulvérulent sous forme liquide aqueuse comprenant une base aqueuse incluant un composant volatile contenant de 20 à 70% en poids d'un constituant consistant en un alcool monohydrique en C₁-C₄, de 0,1 à 5% en poids d'un agent gélifiant et de 5 à 20% en poids d'un constituant pulvérulent, le constituant pulvérulent comprenant au moins une microparticule sphérique acceptable du point de vue cosmétique et un amidon, composition dans laquelle les proportions sont exprimées par rapport au poids total de la composition du gel, et dans laquelle la composition du gel est liquide quand on l'applique à la peau, et, après application, sèche sur la peau pour former une poudre.

2. La composition de gel selon la revendication 1, dans laquelle l'amidon et les micro sphères sont présents en des proportions comprises entre 5:1 et 1:5 (amidon/microsphères), de préférence entre 3:1 et 1,5:1.

3. La composition de gel selon la revendication 1 ou 2, comprenant une microparticule sélectionnée dans le groupe consistant en les produits suivants: nylon, silice, silicone résine, polyéthylène, polyuréthane, et polymères ou copolymères d'acrylate ou de méthacrylate.

4. La composition de gel selon la revendication 1 ou 2, dans laquelle le diamètre de la dimension moyenne des particules des microparticules est de 1 à 20µ.

5. La composition du gel selon l'une quelconque des revendications 1-4, dans laquelle le constituant pulvérulent comprend des microparticules de polyuréthane

6. La composition du gel selon l'une quelconque des revendications précédentes dans laquelle le constituant pulvérulent comprend des microparticules de résine de silicone.

7. La composition de gel selon l'une quelconque des revendications 1-6, qui comprend un agent gélifiant sélectionné dans le groupe consistant en les produits suivants: carbomère, hydroxypropylméthylcellulose, hydroxyéthylcellulose, hydroxy propylguar, hydroxypropylcellulose, amidon de pomme de terre modifié et des polymères croisés à base d'acrylates/alkyl acrylates en C₁₀-C₃₀.

8. La composition de gel selon l'une quelconque des revendications 1 à 7, dans laquelle l'amidon est sélectionnée dans le groupe consistant en l'amidon de tapioca, l'amidon de maïs, l'amidon de riz, l'amidon de pomme de terre et l'amidon de blé.

9. La composition de gel selon la revendication 8, qui comprend une base aqueuse; et un polymère croisé à base d'acrylates/alkyl acrylates en C₁₀-C₃₀, et un constituant pulvérulent comprenant des microparticules sélectionnées dans le groupe consistant en les microparticules de polyuréthane, microparticules de résine de silicone et leurs combinaisons avec du tapioca ou de l'amidon de maïs.

10. La composition de gel selon la revendication 8, qui comprend 10-90% en poids d'une base aqueuse; 0,1 à 5% en poids d'un polymère croisé à base d'acrylates/alkyl acrylates en C₁₀-C₃₀, dans laquelle les proportions précitées sont exprimées par rapport au poids total de la composition de gel, le constituant pulvérulent comprenant des microparticules sélectionnées dans le groupe consistant en les microparticules de polyuréthane, microparticules de résine de silicone et leurs combinaisons avec un amidon.

11. La composition de gel selon la revendication 10 dans laquelle le constituant pulvérulent comprend 0,1-5% en poids de microparticules polyuréthane, 0,1-5% en poids de microparticules de résine de silicone, et 1-20% en poids d'amidon de tapioca, les proportions précitées étant exprimées par rapport au poids total de la composition de gel.

12. La composition de gel selon l'une quelconque des revendications 1-7 dans laquelle le constituant pulvérulent comprend des microparticules sphériques sélectionnées dans le groupe consistant en les produits suivants: microparticules de polyuréthane, microparticules de résine de silicone et leurs combinaisons, ainsi qu'un amidon sélectionné dans le groupe consistant en le tapioca et l'amidon de maïs.
